# EUROPEAN PATENT APPLICATION

(11) **EP 1 965 326 A2**
(43) Date of publication of application: **03.09.2008**
(21) Application number: 08005920.7
(22) Date of filing: 28.11.2000
(51) Int. Cl.: G06F 19/00

(54) **Support method, quality control method, and device therefor**

(30) Priority: 30.11.1999 JP 34108599
(62) Divisional of application: 00310545.9
(71) Applicant: SYSMEX CORPORATION, Kobe-shi, Hyogo 651-0073 (JP)
(72) Inventor: Okuno, Ken'ichi, Kakogawa-shi Hyogo (JP); Morihara, Hiroyuki, Kobe-shi, Hyogo 651-0073 (JP); Yamaguchi, Tadayuki, Hyogo-ku Kobe-shi Hyogo (JP); Sugiyama, Tomomi, Kobe-shi Hyogo (JP)
(74) Representative: Paget, Hugh Charles Edward

(57) **Abstract**

The present invention quickly resolves troubles in an analysis device and to perform effective external quality control. An analysis device 2 and a control device 1 are connected by a network 3. Error data and sample data taken from a measurement of a quality control substance are transmitted from the control device 1 to the analysis device 2. The analysis device 2 is made to be remotely operable from the control device 1 and when troubles arise repair from the control device 1 is possible. The control device 1 analyzes sample data, and provides the analysis results to a web page. The analysis device 2 accesses the web page using a WWW browser, and can perform external quality control in real time.

## Description

### BACKGROUND OF THE INVENTION

### Technical Field

The present invention relates to technology for quality control and support of an analysis device.

### Description of Related Art

Blood tests and other forms of clinical testing require that specimens such as blood and urine be analyzed for a variety of test items. Measurement is performed by analysis devices that employ measurement methods suited to those items to be analyzed. Analysis devices have sophisticated mechanisms that allow them to measure, with a high degree of sensitivity, extremely low concentrations of a substance, and to analyze a small amount of specimen for 10 or more items. To maintain the accuracy of the test results, monitoring is performed of the operations of the mechanism components. When troubles occur in the operations of these mechanism components, an analysis device gives a warning to that effect, alerting the user to trouble therein. In such cases, a user will resolve the trouble by following the operating manual or by, for example, calling a support center, explaining the circumstances, and following the instructions of the technician. When a user cannot resolve trouble by himself, the support center sends a technician to do so.

In diagnostic tests, mechanical check of the analysis device is insufficient for good control of test results to analyze these types of biological specimens, quality control is undertaken wherein measurements are taken of the same or similar biological specimen, which serves as a quality control substance, and those measurement results are monitored.

In one quality control method, the same quality control substance is measured by the same analysis device every day, and monitoring is performed of whether stable measurement results are being obtained (internal quality control). In another quality control method, monitoring is performed of whether measurement results are being obtained that are similar to measurement results at the same type of analysis device used outside that laboratory (external quality control).

However, in order to perform external quality control, the same quality control substance has to be sent from a statistics center to each laboratory; the quality control substance has to be measured at each laboratory; those measurement results (hereinafter "sample data") have to be sent from each laboratory to the statistics center; and the sample data has to be analyzed by the statistics center. The laboratories first learn of the analysis results only after the statistics center sends them. It usually takes one to two months between the time the quality control substance is sent out and the analysis results are returned; because a statistics center must wait until the number of sample data sets to be sent out reaches a set number.

The first problem the invention aims to solve relates to measurements taken when trouble occurs. Because today's analysis devices are operated under the control of sophisticated programs, there are an increasing number of cases where a user is unable to resolve the trouble himself. When this happens, the user has to wait until a technician comes and treats the problem.

If the trouble requires change of or adjustment to an analysis device component, then the only option is to wait for a technician. But there are times when for reasons other than this sort of trouble that users are unable to resolve the trouble themselves. There appear to be many cases where a user should be able to resolve the trouble himself, but is unable to do so, as when a user can't adequately explain the trouble in the analysis device, or when a user cannot properly operate the analysis device in the manner necessary to resolve the trouble.

During an analysis device in the laboratory having trouble, patient tests results cannot be reported to a doctor making a diagnosis. For specimens with low preservability, such as blood, measurements taken the following day will result in test results with lower accuracy, meaning a specimen has to be taken from the patient again.

The second problem the invention aims to solve is that, as described above, for external quality control there is no choice but to wait for the analysis results from the statistics center to confirm accuracy; usually the external quality control is carried out only once a year, and just three or four times a year at the most.

Quality control should be carried out daily before specimen measurement, to improve the reliability of the measurement data. In other words, when the quality control sample data falls outside a predetermined range, the analysis device is insufficient condition, and specimen measurement should not be performed until the analysis device is adjusted so that the sample data falls within that predetermined range. With the external quality control in use today, however, the analysis results come back one or two months after the measurement, meaning that they are used for no other reason than to confirm after the fact the device status at the time of the measurement.

When blood and other substances that denature with the passage of time are the subject of analysis, the condition of the quality control substance used in sample data measurements must be at the same level for each laboratory participating in the external quality control. But when quality control substances are sent out to laboratories for collecting sample data, there is an inevitable tendency for the measurements to be performed on differing days. Thus, because the condition of the quality control substance that is the basis for the sample data differs, the reliability of the analysis results suffers.

### SUMMARY OF THE INVENTION

It is an object of the present invention to present technology making possible effective external quality control with fast, precise resolution of troubles in an analysis device.

In order to solve the above first problem, an aspect of the present invention presents a support method employed in an information terminal connected to analysis devices via a network, the support method comprising: collecting predetermined historical information showing the operational history of the analysis devices from the analysis devices via the network; storing collected historical information for each analysis device; and outputting collected historical information in response to an instruction by the operator of the information terminal.

Communication between the information terminal and the analysis devices is performed through a dedicated NTT line, the Internet or the like. The operational history of each analysis device can be seen by support personnel at, for example, a support center, and this can prevent analysis devices from being down and can facilitate repair work. Collecting historical information by SMTP has the advantage of allowing for easy expansion of the system over a network, since SMTP is usually not subject to the restrictions of firewalls and the like.

In this support method employed in an information terminal, it is preferable to operate the analysis device from the information terminal via a network.

Support personnel in the support center can operate the analysis device while looking at the analysis device operational history stored on the information terminal, and remote support personnel can quickly resolve the trouble without having to travel to the laboratory, leading to a significant reduction in down time.

Furthermore, good use can be made of a user support method wherein error determination parameters are prepared; predetermined error information is extracted from the historical information; based on the error determination parameters, an error history is created; and error history and the analysis device are correlatively stored.

For example, error level is determined based upon how many times the same occurrence occurred in one day. Along with error type, error message, date and time, and other error history, error level is correlated with analysis device and is used in forecasting and solving trouble.

To solve the above first problem, another aspect of the present invention presents a support method employed in an analysis device connected to a predetermined information terminal via a network, wherein predetermined historical information showing the operational history of the analysis device is transmitted at a predetermined timing to the information terminal via the network.

For example, in the shutdown processing of an analysis device the operational history for that day is sent to the information terminal. The predetermined information terminal performs the same function as the information terminal in the above first aspect of the invention.

In the above support method used in an analysis device, it is preferable to accept operation from a predetermined information terminal via a network.

Even if the information terminal is in a distant support center, accepting manipulation from that information terminal allows for the fast resolution of troubles.

In order to solve the above second problem, another aspect of the present invention presents a quality control method employed in an information terminal connected to analysis devices via a network, wherein:
A: sample data that the analysis devices measure predetermined quality control substance is received via a network;
B: the received sample data is stored;
C: the stored sample data is analyzed for each analysis device and each quality control substance; and
D: the analysis results of the sample data received within a predetermined timeframe are provided to the analysis devices.

Communication between the information terminal and the analysis devices is performed through a dedicated NTT line, the Internet or the like. An analysis device performs a daily measurement of a quality control substance, such as control blood, and transmits the measurement data to the information terminal. The information terminal stores the measurement data sent from analysis devices and analyzes the stored measurement data for each analysis device and each quality control substance. Each time the information terminal receives sample data from an analysis device it performs a new analysis.

In order to ensure that the analysis results come from quality control substances having similar levels of condition, the analyses can be of sample data from measurements performed within a certain timeframe, for example, within 24 hours of reception. When an analysis device requests analysis results, the latest analysis results are provided in real time. In the present invention it is preferable to use SMTP in the communications, as it is not usually subject to the restrictions of firewalls and the like.

In order to solve the above second problem, another aspect of the present invention presents a quality control method employed in analysis devices connected to a predetermined information terminal via a network, wherein:
A: sample data that predetermined quality control substances are measured is transmitted to the information terminal via the network;
B: the analysis results of the sample data is requested to the information terminal;
C: analysis results based on the sample data that the information terminal has collected from the analysis devices within a predetermined timeframe is received from the information terminal; and
D. the analysis results are output.

With this method, in the above quality control method employed in an information terminal, the results that an information terminal analyzes is output to a display of the analysis device or to a printer or other output device. A user refers to the output results when performing quality control of his analysis device.

Another aspect of the present invention also presents a computer-readable storage medium on which is recorded a program for executing the support method used in an information terminal or analysis device as described above. Conceivable recording media include floppy disks, hard drives, semiconductor memory, CD-ROMs, DVDs, and MO disks.

Another aspect of the present invention also presents a control device connected to analysis devices via a network, comprising: reception means for receiving from the analysis devices predetermined historical information showing the operational history of an analysis device; storage means for storing historical information for each analysis device; and output means for outputting historical information in response to an instruction by an operator.

This has the same operational effect as the above support method used in an information terminal.

Another aspect of the present invention presents an analysis device connected to a predetermined information terminal via a network, comprising transmission means for transmitting predetermined historical information showing operational history to the information terminal via the network according to a predetermined timing.

This has the same operational effect as the above support method used in an analysis device.

Another aspect of the present invention also presents a control device connected to analysis devices via a network, comprising: reception means for receiving sample data that the analysis devices measure predetermined quality control substances; storage means for storing received sample data; analysis means for analyzing the stored sample data for each analysis device and each quality control substance; and providing means for providing to the analysis device analysis results of sample data received within a predetermined timeframe.

This has the same operational effect as the above support method used in an information terminal.

Another aspect of the present invention also presents an analysis device connected to a predetermined information terminal via a network, comprising: transmission means for transmitting sample data that predetermined quality control substances are measured to the information terminal via the network; request means for requesting to the information terminal for analysis results of the sample data; reception means for receiving from the information terminal the analysis results of the sample data that the information terminal has collected from the analysis devices within a predetermined timeframe; and output means for outputting received analysis results.

This has the same operational effect as the above support method used in an analysis device.

### BRIEF DESCRIPTION OF THE DRAWINGS

Fig. 1 is an example of an overall block diagram of the remote support system relating to the first embodiment.
Fig. 2 is a block diagram showing function and constitution.
Fig. 3 is an example of the processing flow in a remote support system.
Fig. 4 is a flowchart showing one example of the flow of the main processing performed by a control device.
Fig. 5 is a flowchart showing one example of the flow of the support processing performed by a control device.
Fig. 6 is a flowchart showing one example of the flow of the QC processing performed by a control device.
Fig. 7 is a flowchart showing one example of the flow of the main processing performed by an analysis device.
Fig. 8 is an example of an error information selection screen.
Fig. 9 is a display example of error history.
Fig. 10 is a display example of program history.
Fig. 11 is a display example of number of times operated.
Fig. 12 is an example of error determination pattern.
Fig. 13 is an example of error determination table.
Fig. 14 is a display example of a web page created by QC processing (menu screen).
Fig. 15 is display example of a web page created by QC processing (analysis results)
Fig. 16 a display example of a web page created by QC processing (analysis results).
Fig. 17 is overall block diagram of a remote support system relating to another embodiment.
Fig. 18 is overall block diagram of a remote support system relating to another embodiment.
Fig. 19 is a conceptual diagram of data sent to a control device by an analysis device.
Fig. 20(a) is when the past 24 hours are subject to analysis, and Fig. 20(b) is when the past 48 hours are subject to analysis.
Fig. 21(a) is conceptual diagram of today's analysis processing, and Fig. 21(b) is conceptual diagram of the previous day's analysis processing.
Fig. 22 is flowchart showing one example of the flow of collection processing.
Fig. 23 is flowchart showing one example of the flow of today's analysis processing.
Fig. 24 is flowchart showing one example of the flow of the previous day's analysis processing.

### DETAILED DESCRIPTION OF THE PREFERRED EMBODIMENTS

The support method and quality control method of the present invention will be explained in detail with reference to the figures.

### First Embodiment

### [SUMMARY]

This embodiment will be explaining using as an example a remote support system that is a realization of the methods of the present invention. This remote support system is constituted by an analysis device owned by a laboratory (i.e., a user) and a control device of the party providing the system, said devices being interconnected by a dedicated network.

The analysis device transmits predetermined historical information according to a predetermined timing to the control device over the network. Contained in the historical information are operational information showing the operational conditions of the analysis device and measurement data. The operational information comprises error information, number of times operated, operation program, set-up parameters and the like for each analysis device. The measurement data comprises specimen data from patient and sample data from a quality control substance.

The control device performs support processing, collecting historical information from each analysis device, editing the historical information, according to the contents, for each analysis device, and storing the information, and performs Quality Control (QC) processing.

### A. Support Processing

The control device edits operational information from collected historical information and stores that information. The control device also analyzes error content based on operational information, and if there is a significant error it displays that error. Because a technician can review at the control device the historical information of the analysis device where the error arose, he can sufficiently understand the conditions of the machine without needing a detailed explanation from the user, and can work on finding the cause of the trouble.

In addition, the analysis device is provided with the capability to remotely operate the analysis device. Therefore, a technician does not actually have to go to the laboratory, but can work on the analysis device directly from the control device. Furthermore, the control device can analyze error information, predict when an analysis device will have trouble, and take measurements to prevent trouble before it occurs.

### B. QC Processing '

A control device 1 analyzes sample data from a quality control substance measured at each analysis device 2 for each type of analysis device 2 and each type of quality control substance. Each time the control device 1 receives sample data, it updates the analysis results for the same type of sample data at the same type of machines, and provides the latest analysis results on a web page. By accessing this web page, the analysis device 2 can acquire the latest analysis results. When an analysis device attempts to access the web page, the control device authenticates the authentication information input by the analysis device. In this manner, soon after measuring a quality control substance, a user can confirm in real time the very latest analysis results for the quality control substance.

### [CONSTITUTION]

### (1) Overall Constitution

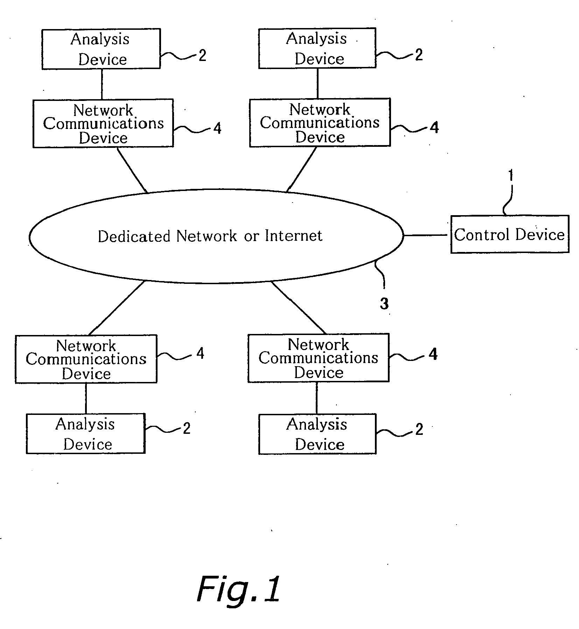

FIG. 1 is one example of an overall block diagram of a remote support system according to the first embodiment. In the remote support system according to this embodiment, the control device 1 and the analysis devices 2 are interconnected over a dedicated network 3.

The analysis device 2 is interconnected with the dedicated network 3 via a network communications interface 4. Possible analysis devices include hemanalysis and urinalysis devices. Personal computers, workstations and the like can be used as the control device 1. Dial-up routers and modems can be used as the network communications interface 4.

One example that could be given of a dedicated network 3 would be a dedicated telephone line that the provider of this system is able to use exclusively, through a contract with the company providing the telephone line. Other types of networks than dedicated networks can be used, such as the Internet and intranets and LANs.

### (2) Control Device

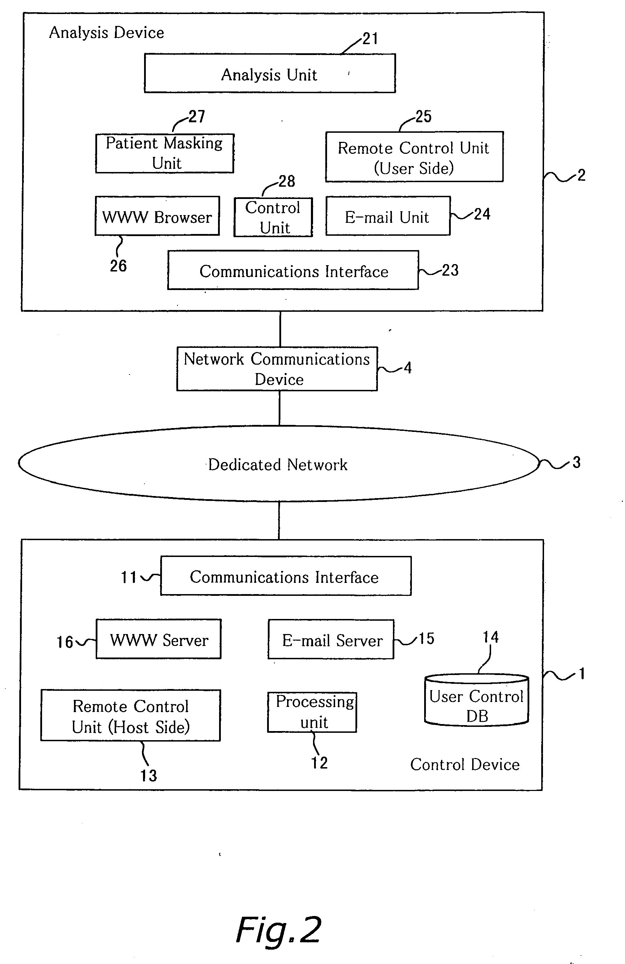

FIG. 2 is a block diagram showing the functions and constitution of the control device and the analysis device.

The control device comprises a communications interface 11, a processing unit 12, a user control database 14, an e-mail server 15, a WWW server 16 and a remote control unit (host side) 13.

The communications interface 11 establishes a connection with analysis devices.

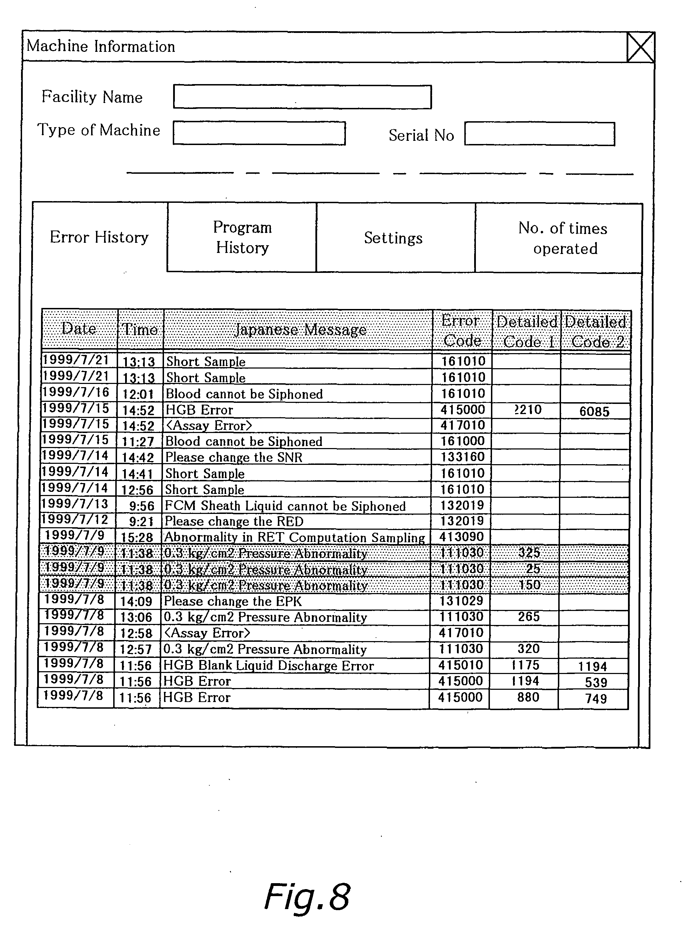

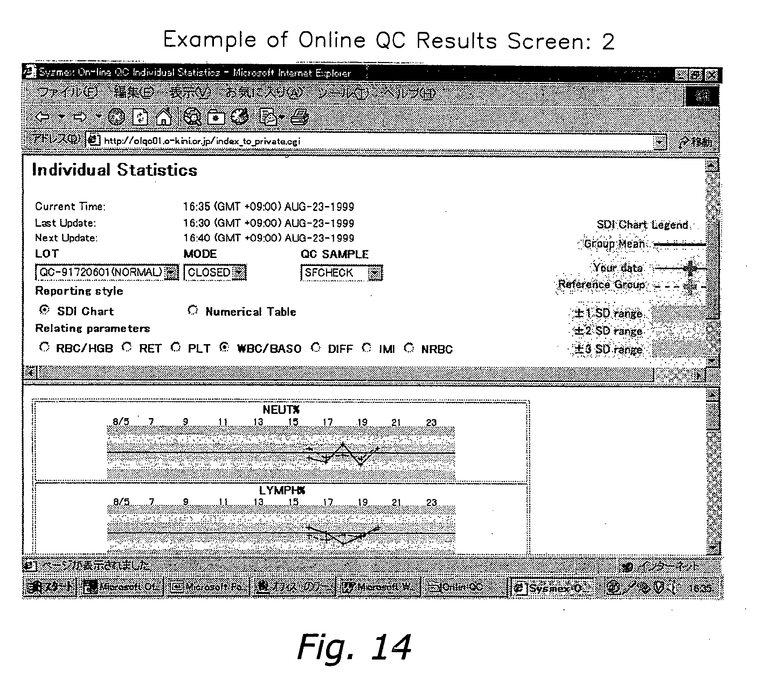

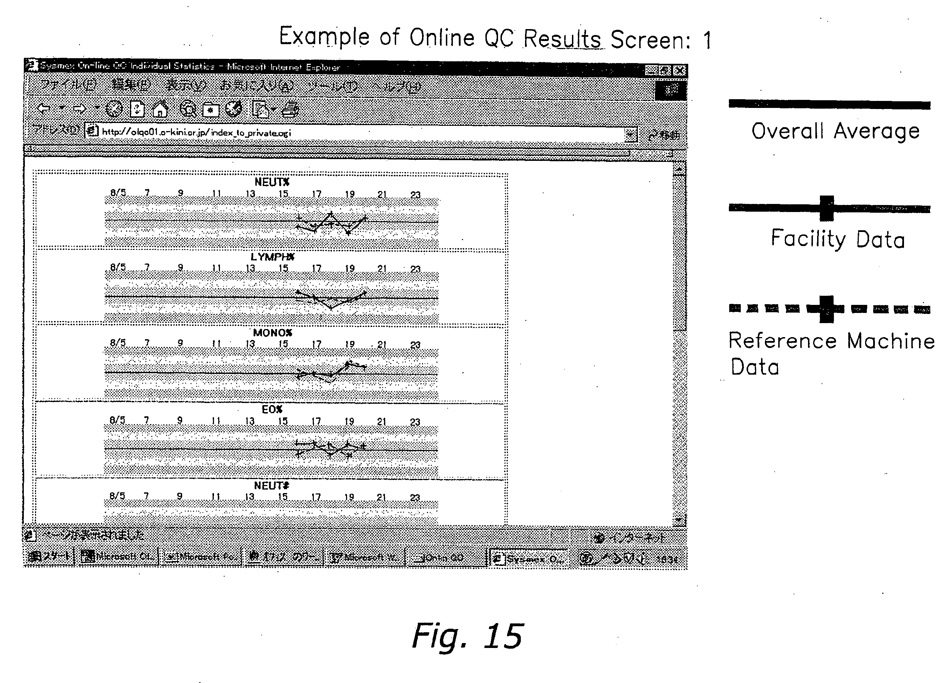

The processing unit 12 performs support processing and QC processing, using the user control database 14. The support processing displays predetermined error history at the control device, making it possible to find the cause of the trouble. FIGS. 8 through 11 show display examples of the error history output by the processing unit [12]. The QC processing makes possible real time external quality control at the analysis device. FIGS. 14 and 15 show examples of web pages for analysis results created by the QC processing. These examples will be discussed in detail below.

The user control database 14 stores at each analysis device error history, number of times operated, QC data, log information and the like.

The e-mail server 15 receives historical information and sample data from analysis devices through SMTP. The communications protocol is not limited herein to SMTP, but SMTP has the advantage of facilitating future expansion of this system, due to the fact that it is usually not subject to the restrictions of firewalls and the like.

The WWW server 16 provides a WWW browser on the analysis device with the web pages that processing unit 12 has created.

The remote control unit (host side) 13, by being linked with the remote control unit (user side) on the analysis device 2, makes possible the remote operation of the analysis device 2. Because the two units are interlinked, the analysis device can be logged onto remotely, the window displayed at the analysis device is displayed at the remote control unit (host side) 13, and the analysis device can be operated pursuant to the operations input from the remote control unit (host side) 13.

### (3) Analysis Device

An analysis device 2 has an analysis unit 21, a communications interface 23, an e-mail server 24, a remote control unit (user side) 25, a WWW browser 26, a patient masking unit 27 and a control unit 28.

The analysis unit 21 measures the quality control substances and generates sample data.

The communications interface 23, as with the communications interface 11 in the above control device 1, establishes a connection.

The e-mail server 24 sends historical information showing the operational history of an analysis unit 21 and sample data to the control device using SMTP.

The remote control unit (user side) 25, by being interlinked with the remote control unit (host side) 13, makes possible the operation of the analysis device 2 from the control device 1.

The WWW browser 26 acquires web pages from the control device based on instructions from a user.

The patient masking unit 27 ensures that when the analysis device 2 is operated from the control device 1, patient information is not displayed at the control device.

The control unit 28 controls the operations of the analysis unit 21 and of the constituent elements of the user terminal 22.

### PROCESSING FLOW

An explanation will be given of the processing performed by the control device and analysis device in a remote support system.

### (1) Processing Flow of the Overall System

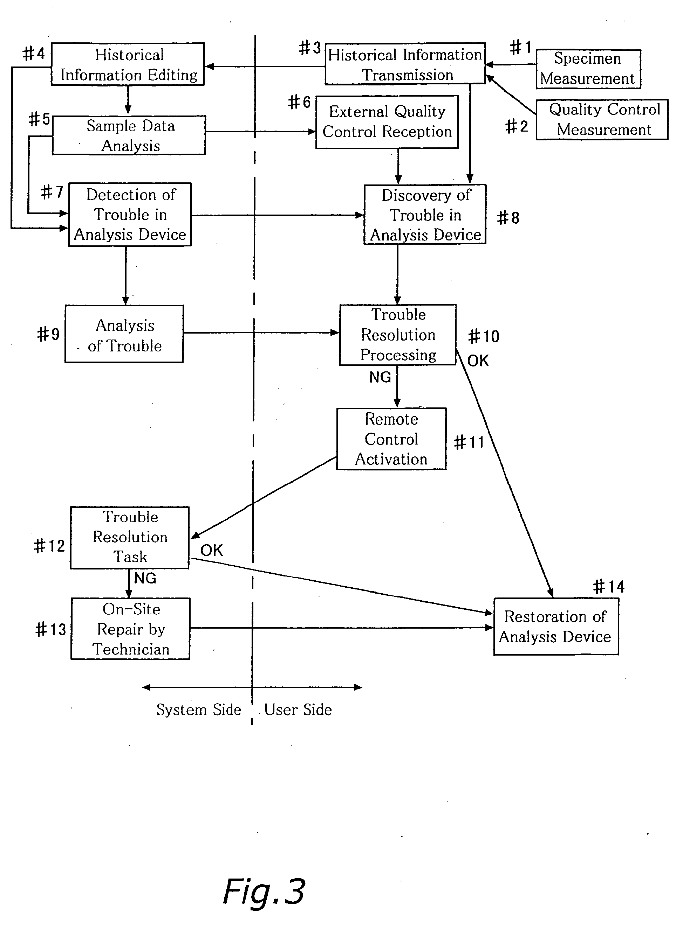

An explanation will be made in detail of the processing flow of the overall system. FIG. 3 is an explanatory diagram showing an example of the flow of user support in a remote support system.

The analysis device 2 performs standard specimen measurement (#1), and its operational information is transmitted to the control device 1 according to a predetermined timing (#3). The transmission is made in real time if the operational information contains error information or other urgent information. The transmission is made when the analysis device is shutdown if the operational information is not urgent, such as number of times operated and specimen measurement results. Error information is also displayed at the analysis device 2, too, and the user discovers that there is trouble at the analysis device 2 (#8).

The control device 1 classifies operational information sent from the analysis device 2 according to type and stores this in the user database 14 (#4). When there is major error information in the stored operational information, or when there are other indications that a predetermined major error will occur, such as when there is minor error information, but the error occurs frequently or when error conditions are worsening, the trouble the analysis device is having is detected based on certain settings (#7).

When the analysis device 2 measures a quality control substance, unlike standard specimen measurement results, the sample data is transmitted to the control device 1 in real time (#3). The analysis device 2 reads a barcode affixed to the measurement specimen container, determines whether that specimen is a quality control substance or not, and based on that determination, transmits the sample data. The control device 1 takes the new sample data and updates the analysis results (#5).

The user, after measurement of the sample data, acquires the analysis results that the control device 1 has analyzed (#6) and confirms the external accuracy. The control device 1 updates the web pages in accordance with updates to the analyzed data. The analysis device 2 accesses a web page, and when access is authenticated, the latest analysis results and the sample data are provided on the web page.

In this manner, a user can quickly confirm not just internal quality control results, but external quality control results as well, and can discover malfunctions in an analysis device in real time (#8).

The control device 1 analyzes quality control data. If the quality control results fall outside of a predetermined range, or if a worsening of the quality control data is anticipated, trouble in the analysis device 2 is detected based on predetermined settings (#7). For example, data is trending away from median values. If trouble at the analysis device 2 is detected at the control device 1, the user is notified to that effect (#8).

If trouble at the analysis device is discovered (#8), the user performs processing to resolve the trouble (#10). The control device 1 analyzes the trouble from the edited operational information of that analysis device 2 (#9), and provides the user with the most suitable information for solving the trouble.

If it is difficult for the user to resolve the trouble himself, the user activates the remote control unit of the analysis device (#11). A technician at the support center remotely operates the analysis device 2 and performs task for resolution of the trouble via the remote control unit of the control device (#12). Thereupon the screen of the analysis device and the screen of the control device are linked. In this manner, with regard to troubles that can be resolved through operation of the analysis device, even a complicated problem can be resolved through remote operation from the support center (#14). For troubles that cannot be resolved thus, a technician would go and make repairs (#13, #14).

### (2) Flow of Processing in Control Device

Next, the flow of processing that the control device 1 performs in a remote support system will be explained in detail.

### (2-1) Collection Processing

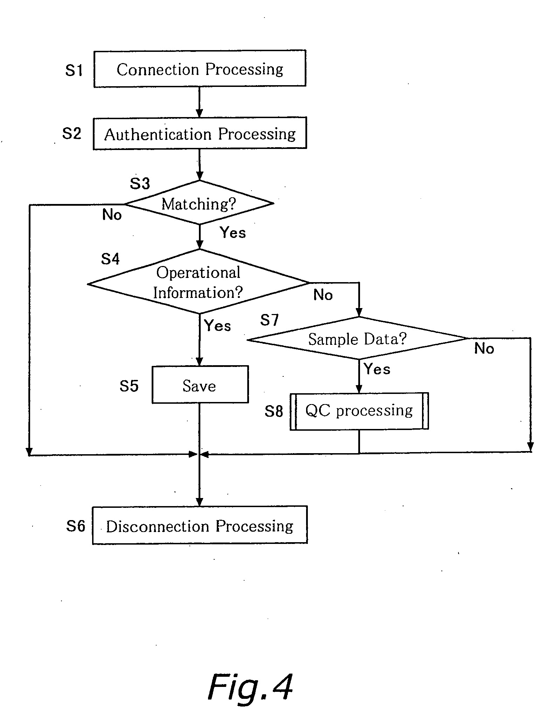

FIG. 4 is a flowchart showing one example of the flow of the main processing that the control device 1 performs. In the main processing, the control device 1 collects historical information from the analysis device 2, and if it is operational history, stores it, and if it is sample data, performs QC processing. The following processing commences by means of the dial-up router from the analysis device 2.

In Step S1, the communications interface 11 performs connection processing to establish a connection with the analysis device 2.

In Step S2, the processing unit 12 performs predetermined authentication processing. In other words, it determines whether the authentication information sent from the analysis device 2 matches the user information in the user database.

In Step S3, the processing unit 12 performs processing according to the authentication results. If it determines that the authentication information matches, operation proceeds to Step S4. If it doesn't match, then the connection is cut or other similar processing is performed.

In Step S4, the e-mail server 15 receives data from the analysis device 2. The processing unit 12 determines whether the received data is predetermined operational information or not. Operational information is predetermined information other than sample data, for example, error data, number of times operated, program history, and set-up information. If the answer is "yes," then operations proceed to Step S5; if "no," operations proceed to Step S6.

In Step S5, the processing unit 12 temporarily saves the received operational information. This is use for in the support process, which is discussed below. In support processing, for example, operational information from each analysis device 2 until 00:00 midnight, when the date changes, is stored; when the time reaches 00:00, operational history is created based on the operational information received that day.

In Step S6, communications interface 11 severs the connection with the analysis device 2.

In Step S7, the processing unit 12 determines whether the received data is sample data from an measurement of a quality control substance. If it determines "yes," then operations proceed to Step S8, and proceed to the QC, which is discussed below. In other words, sample data, including received data, is analyzed, and the web page for each analysis device is updated. If the answer is "no," the operations proceed to the above-described Step S6, and the connection is severed.

### (2-2) Support Processing

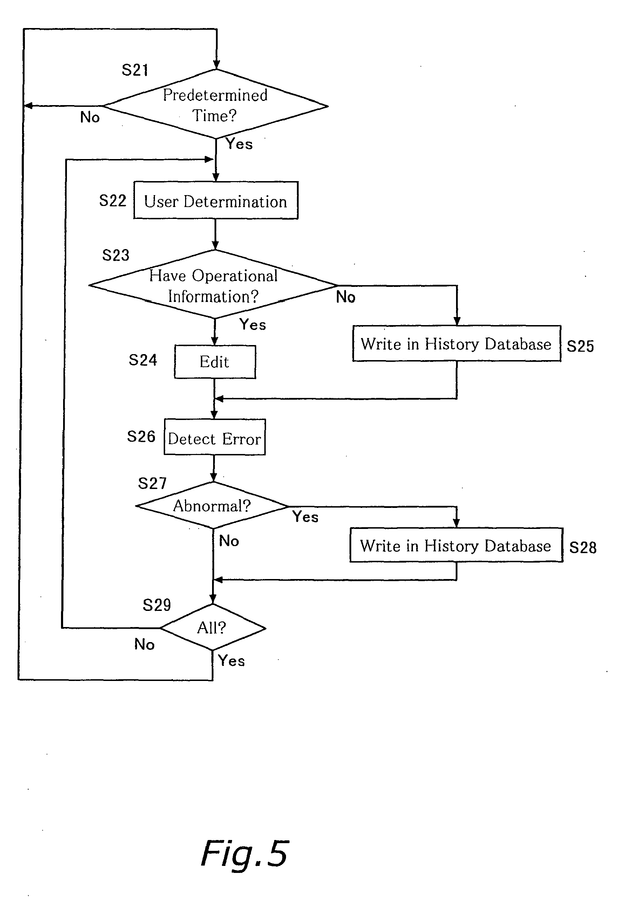

FIG. 5 is a flowchart showing one example of the flow of support processing that the control device 1 performs independently of the main processing. Every time the date changes, the control device 1 edits the operational information received that day and writes that to the history database.

In Step S21, the processing unit 12 is waiting for a predetermined time, for example, 00:00.

In Step S22, the processing unit 12 determines which analysis device 2 among those registered in the user control database 14. is the subject user.

In Step S23, the processing unit 12 determines whether it has received operational information showing operating conditions for that date for the subject user. If it determines "yes," operations proceed to Step S24. If it determines "no," then operations proceed to Step S25.

In Step S24, the processing unit 12 edits operational information for each analysis device and each subject matter, and writes this to the history database. For example, it edits error information, number of times operated, operation program, and setting parameters, in separate table format with date and time, and writes this to the history database.

In Step S25, the processing unit 12 writes to the user control database 14 predetermined error information showing that operational information could not be acquired. Possible examples of error information include analysis device name, date, time, error number showing the error that arose, and error message corresponding to error number.

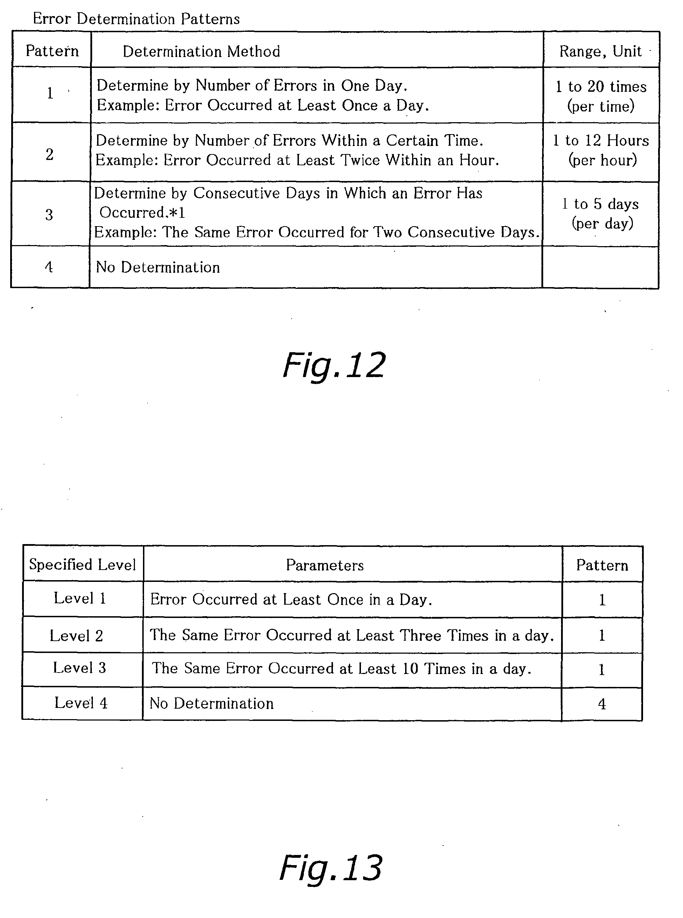

In Step S26, the processing unit 12 searches for a predetermined error based on the error information of the subject user. For example, using the methods for determination shown in FIG. 12, error levels are decided, as in the example shown in FIG. 13, from error type and the frequency with which the same type of error occurs.

In Step S27, the processing unit 12 uses the search results to determine whether or not errors are contained in the operational information of the subject user. Unless the error level is "0", the determination is "Yes." If the determination is "Yes," operations proceed to Step S28; if "No," operations proceed to Step S29 to be discussed later.

In Step S28, the processing unit 12 writes the determined error level to the user control database 14.

In Step S29, the processing unit 12 determines whether Step S23 through Step S28 have been performed for all registered analysis devices 2. If "Yes," operations return to Step S21, that is, it waits for the date to change. If "No," then operations return to Step S22, and chooses another analysis device as the subject user.

### (2-3) QC Processing

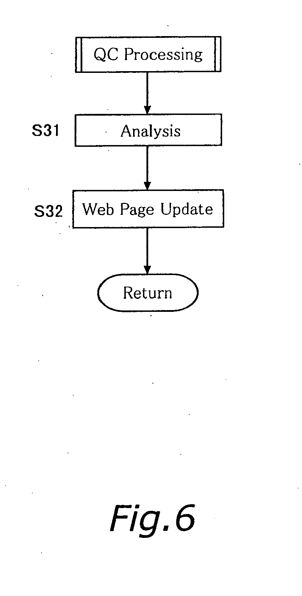

FIG. 6 is a flowchart showing the flow of the QC processing the analysis device 1 performs. In the above-discussed main processing, when the control device 1 receives sample data from any analysis device 2, the control device 1 performs the following QC processing. In other words, it analyzes sample data including newly received sample data, and updates the web page for each analysis device using the new analysis results.

In Step S31, the processing unit 12 analyzes newly receiving sample data including newly received sample data. It is common for different of quality control substances to be used, including some with high values and others with low values, or some with normal values and others with abnormal values, for the same measurement item. When a quality control substance is from biological matter, values will usually differ from lot to lot-that is, the lot number will be different for each time of manufacture. Consideration must also be given to the' measurement mode under which the sample data was collected. Thus the analysis device notifies the control device of such information as the type, lot number, measurement mode of the quality control substance, in a manner to be described later.

Analysis is made for each type of analysis device and for each quality control substance. Because substances like blood, which are easily susceptible to denature over time, are used as an quality control substance, analysis is made for each measurement date, improving the reliability of the analysis results.

Because in the present invention, the latest analysis results are presented in real time, there is the danger that at an early morning hour there will be an insufficient number of sample data sets for measurements taken on a given day, meaning that the reliability of the analysis results will be in question. Therefore, the analyses for measurements taken on a given day will be based on sample data received, for example, within the past 24 hours. This allows the use of sample data that has been collected under the same elapsed-time conditions, and prevents the number of sample data sets from fluctuating markedly because of time of day. When the date changes, the analysis results from the past 24 hours are finalized as the analysis results for that day.

To improve the reliability of the analysis results, it is preferable that cutoff values of mean plus or minus 3SD be used, and that values far outside the normal range not be included in the analysis. When the analysis results are presented in the form of the average value of the sample data, and there is a very small amount of data for a 24-hour period, it would be better to use median value in place of average value.

In Step 532, the processing unit 12 updates the web page for each analysis device based on the new analysis results. Then it returns to the main processing and severs the connection with the user terminal.

It should be noted that the timing for updating the analysis results is not limited to [being based on] time sample data was received, as long as the timing is such that the latest analysis results can be presented to the analysis device. For example, one conceivable alternative would be to update the analysis results when a web page has been accessed from an analysis device. Or, the analysis results may be updated at a predetermined time interval set in consideration of the load being placed on the analysis device.

### (2-4) Other Processing

The analysis device 1 performs other processing in addition to main processing, support processing and QC processing. '

For example, the WWW server 16 provides a web page when the WWW browser on the analysis device has accessed the web page. On this occasion, it is preferable that the analysis device perform authentication processing in the form of an interface program, such as a library or CGI scripts.

Also, the processing unit 12, in response to instructions from the operator of the control device 1, displays error history stored in the user control database 14.

### (3) Processing Flow in the Analysis Device

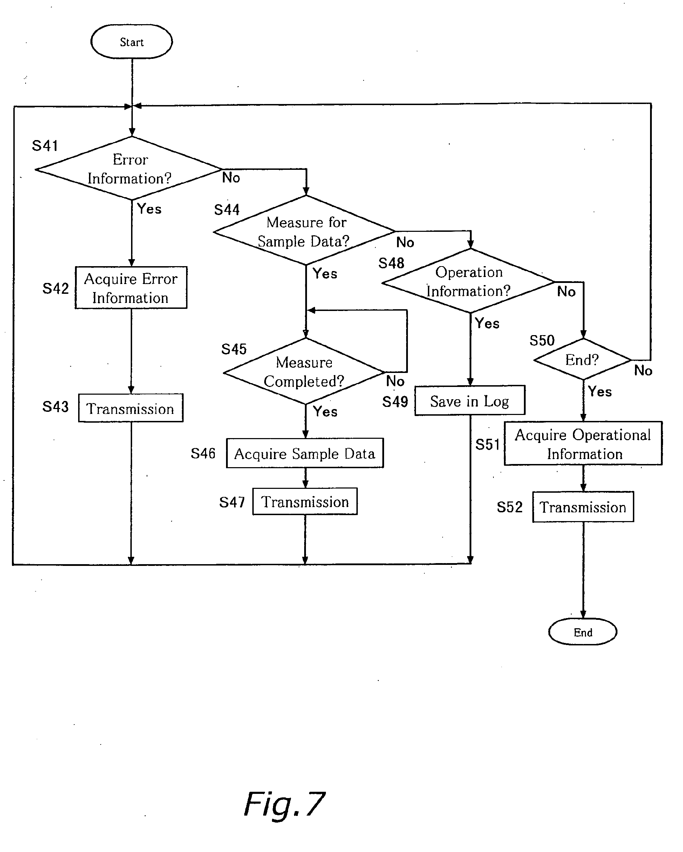

FIG. 7 is a flowchart showing one example of the flow of the main processing performed by the analysis device. The analysis device 2 transmits error information and sample data in real time, and transmits operational information other than error information when the operations of the device end. FIG. 7 shows only the flow according to the present invention. When the analysis device is activated, the following processing commences.

In Step S41, the control unit 28 monitors the operational conditions of the analysis unit 21 and determines whether error information has occurred or not. If it determines "Yes," then operations proceed to Step S41. If "No," then operations proceed to Step S44, explained later.

In Step S42, the control unit 28 acquires error information from the analysis unit 21 and processes it to be data for email. For example, it creates email in which analysis device authentication information and error information is written into the main body of the text.

In Step S43, the control unit 28 activates the e-mail server 24 and transmits the created email. Then operations return to Step S41.

In Step S44, the control unit 28 determines whether sample data is to be collected. If it determines "Yes," then operations proceed to Step S45. If "No," operations proceed to Step S48, explained later.

In Step S45, the control unit 28 awaits completion of the measurement. Upon completion operations proceed to Step S46.

In Step S46, the control unit 28 acquires sample data from the control device 1 and processes it to be data for email. For example, it writes authentication information into the text of the email, and creates an email with the sample dated attached as a file attachment. Other information needed when analyzing sample data may be included in the file attachment, for example, lot number, type of quality control substance, measurement mode, and device ID. Device ID is identification information for the purpose of identifying an analysis device on this system, and is used to prevent sample data from being entered more than once during analysis.

In Step S47, the control unit 28 activates the e-mail server 24 and transmits the created email.

In Step S48, the control unit 28 awaits for operational information showing the operational conditions of the control device 1 other than error information. Operational information other than error information can include number of times operated, operation program, set-up conditions and the like. When operational information arises, operations proceed to Step S52. In all other cases, operations return to Step S41.

In Step S49, the control unit 28 saves in a log the operational information that has arisen.

In Step S50, the control unit 28 determines whether instructions have been given for completion of the analysis device. If it determines "Yes," then operations proceed to Step S49. If "No," then operations proceed to Step S51, described later.

In Step S51, the control unit 28 acquires operational information from the log and processes this to be email data. For example, it creates email in which analysis device authentication information and operational information are written into the text of an email.

In Step S52, the control unit 28 activates the e-mail server 24 and transmits the created email. After that, the control unit 28 terminates operations.

### [EXAMPLE OF OPERATIONAL HISTORY STORED IN THE HISTORY DATABASE BY SUPPORT PROCESSING]

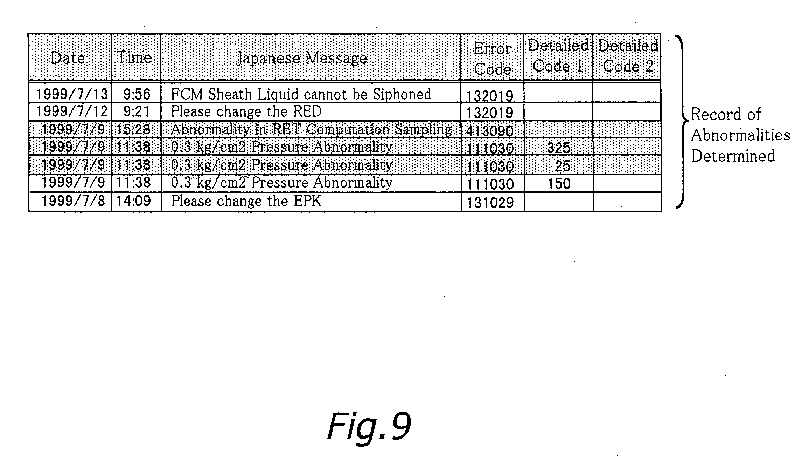

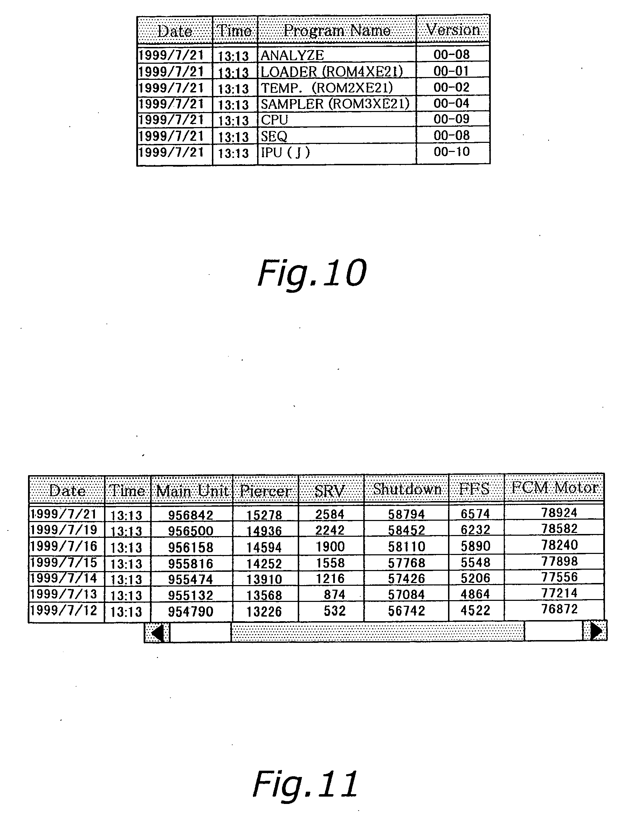

An explanation will be given in detail regarding the operational information stored in the user control database 14 by the support processing described above. FIGS. 8 through 11 show examples of operational information displayed at the control device 1 when a hemanalysis device has been used as the analysis device. FIG. 8 shows an example of an operational information selection screen, FIG. 9 shows an example of error history, FIG. 10 shows an example of program history, and FIG. 11 shows an example of number of times operated.

The operational information selection screen of FIG. 8 accepts selections for error history, program history, settings, or number of times operated. An operator can use this screen to designate analysis device and type of analysis device.

FIG. 9 shows an example of a screen displayed when "error history" has been selected on the operational information selection screen of FIG. 8. Error date and time, error message describing error, error code specifying error, and detailed code 1 and detailed code 2 are displayed. This error history displays, for example, the latest month worth of error history stored in the history database. It is preferable that it be possible to make settings for sort and filter for each field. It is also preferable that records of abnormalities that have a high possibility of being the cause of trouble be displayed in an easily distinguishable reverse display or the like. Record of abnormalities, for example, are records of the above-described error level being above a predetermined value.

FIG. 10 shows an example of a screen displayed when "program history" has been selected on the screen of FIG. 8. In this example, the program name of the program operated at the designated analysis device, the version thereof, and the time and date operated are displayed.

FIG. 11 shows an example of a screen displayed when "number of times operated" has been selected on the screen of FIG. 8. In this example, the number of times that a predetermined unit of the analysis device has been operated is displayed along with the operation date and time.

Although not shown in the figures, when "settings" is selected on the selection screen of FIG. 8, the setting terms for the analysis device are displayed.

### [SPECIFIC EXAMPLE OF WEB PAGE CREATED BY QC PROCESSING]

An explanation will be given of a specific example of a web page created by the control device 1 using the QC processing described above. FIGS. 14 and 15 show examples of web pages created by the processing unit 12. As before, these are examples of displays of analysis results when analyses are made of a quality control substance using a hemanalysis device.

When a WWW browser on an analysis device accesses the control device 1, the window shown in the top half of FIG. 14 is displayed. This window allows the selection of a display style for the analysis results. Here, an SDI chart has been selected as the "reporting style," causing the window shown in FIG. 15 to be displayed.

In FIG. 15, a predetermined graph is displayed for each blood component. This graph is created for each type of analysis device and each quality control substance. This graph is capable of displaying the past month's daily sample data for the accessing user and reference machine data. The reference machine data is sample data from measurements of a predetermined quality control substance taken at an analysis device of the provider of the remote support system. The graph also displays degree of deviation from mean value for each 1SD. The daily analysis results are finalized when the date changes.

In terms of internal quality control, the as is display of these measurement values allows confirmation of the fluctuations in sample data from an analysis device. In terms of external quality control, confirmation is possible of the fluctuations in the sample data from an analysis device against the overall average, using the overall average at the time of taking the sample data, as shown in FIG. 15. By changing the display as he sees fit, a user can make a visual comparison to see how much the sample data of the analysis device deviates from the overall average and the reference machine data. Furthermore, the web pages on FIGS. 14 and 15 are updated immediately after sample data has been submitted. Therefore, a user can perform external quality control for the sample data he has submitted in real time, without a time lag.

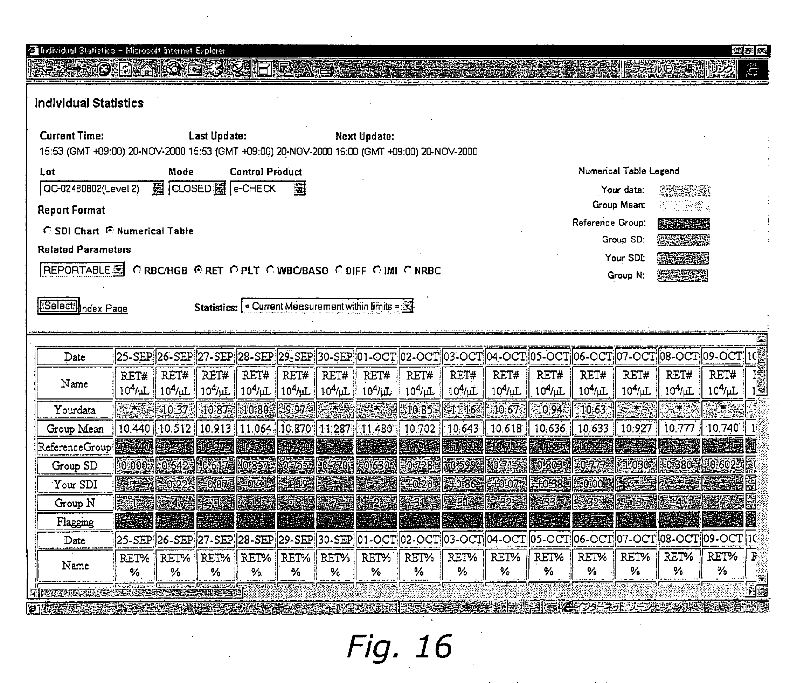

FIG. 16 is another display example of analysis results for an quality control substance. In this example, the measurement values for the user's analysis device, overall average value, and reference machine data are displayed individually. Because there are times when the user wants to make direct comparisons between the measurement values of his own analysis device and the overall average and reference machine data, it is preferable to make it possible to display individually the values in FIG. 15 that are displayed within a chart.

### OTHER EMBODIMENTS

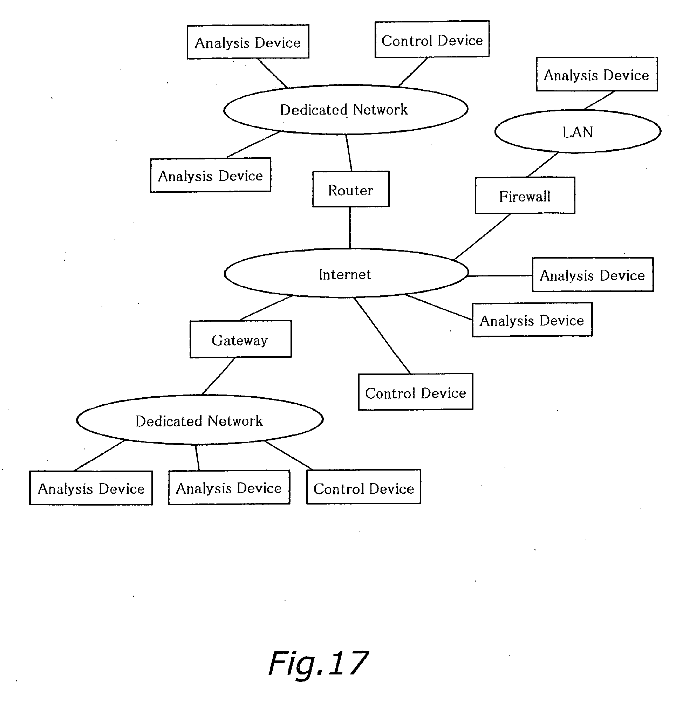

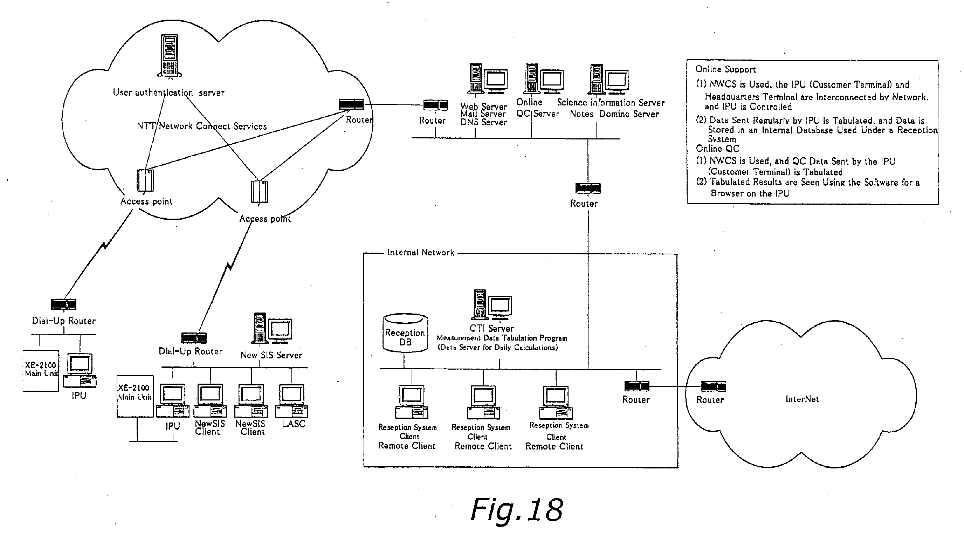

(A) FIGS. 17 and 18 are block diagrams showing other examples of the remote support system. The network linking the user terminal and the analysis device does not necessarily have to be a dedicated network, but may be the Internet or a LAN. However, when the Internet is used, encoding and a stricter authentication system need to be used to heighten security when transmitting information.

It is not necessary for there to be just one control device on the system. For example, separate dedicated networks may be connected by the Internet and routers and gateways, and a control device may be provided for each dedicated network. In addition, a control device can collect predetermined information from analysis devices of a dedicated network, for example analysis devices on the Internet connected via a dedicated network and router, or analysis devices connected to a LAN connected to the Internet via a firewall.

(B) In the above first embodiment, no considerable is given to possible differences in times zones between the control device 1 and the analysis devices 2 and between analysis devices when QC processing is conducted. Therefore, as the second embodiment, an explanation will be given of QC processing in a remote support system having a control device and analysis devices in different times zones.

### (B-1) Operation of the System

Analysis of sample data is conducted in the following way. In the same manner as the first embodiment, data collected in the past 48 hours is analyzed, and those results become real time analysis results. Alternatively, the analysis results for each day are computed by analyzing from among the data collected in the past 48 hours, that data collected during the previous day.

To make it easy for operators of analysis devices in each time zone to confirm analysis results, analysis results are correlated with those time zones (i.e., local time) and so inscribed.

However, when the reference time for analysis is set as local time, the reference time will differ from time zone to time zone, and thus analyses have to be conducted for each time zone. This means that there will be 24 different analysis results across the world for a single date, making operation of the system complicated. On top of this, there are countries that have more than one time zone, and group hospitals that are located across more than one time zone.

On the other hand, when one of the time zones is the basis for the analysis reference time, without regard to local time, the differential between local time and reference time becomes a problem. For example, confusion will result if the date of QC processing changes in the middle of the analysis.

For this reason, to ensure that the analysis results for a given day are the same for all time zones, the analysis results for that day are computed with sample data having the same measurement date (local time) according to each time zone.

### (B-2) Base

In consideration of the above, in this embodiment, the reference time for the control device 1 is made to be the world's most advanced time, namely, GMT (Greenwich Mean Time) + 12 hours. In the explanation below, the reference for time of day is the time of day of the time zone in which the control device 1 is located, in this instance, GMT + 12 hours. Each analysis device 2 transmits to the control device 1, along with the sample data, the meausurement time and date in the time zone in which it is located. The control device 1 conducts analysis of the sample data based on sample data having an measurement time and date within the past 48 hours. The reason for analyzing sample data for the past 48 hours rather than the past 24 hours is to ensure that there will be a sufficient number of sample data sets N that will form the basis of the analyses.

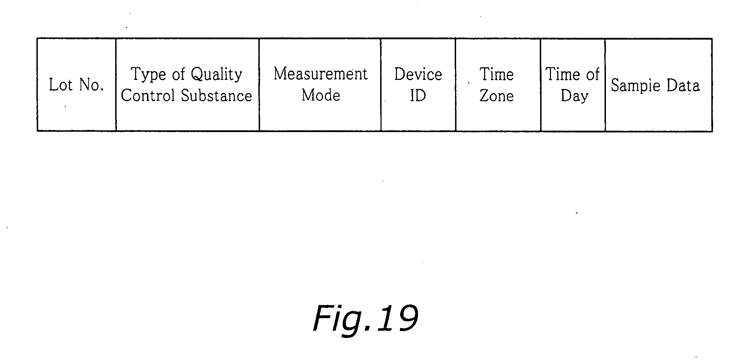

FIG. 19 is a conceptual diagram of data transmitted from the analysis device 2 to the control device 1. Included in this data are lot number, type of quality control substance, measurement mode, device ID, time zone, time of day, and sample data. Except for time zone and time of day, all other data is the same as in the first embodiment. For time zone, the time zone in which the analysis device 2 is located is given. For time of day, the measurement date and time in the time zone in which each analysis device is located is given. The control device 1 conducts the QC. processing to be discussed later based on sample data having measurement date and time within 48 hours of the time of day in the time zone in which the control device is located.

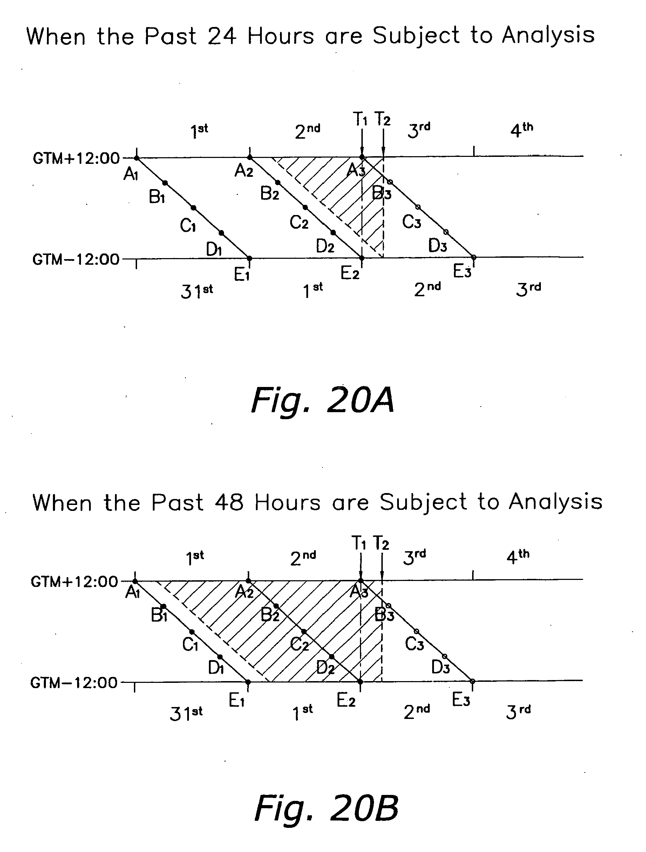

FIG. 20(a) is an explanatory diagram showing there being an insufficient number of sample data sets N received in the past 24 hours. To facilitate the explanation, let us suppose that analysis devices A, B, C, D, and E, located in different times, transmit sample data daily at the time of 00:00. Analysis device A is in the GMT + 12 hours time zone. Analysis device E is in the GMT - 12 hours time zone, and analysis devices B, C, and D are in time zones in between. The control device is in the GMT + 12 hours time zone.

In FIG. 20, sample data with a date of X are indicated as Aₓ, Bₓ, etc. For example, A₁, A₂, and A₃ represent sample data from analysis device A dated the 1^{st}, 2^{nd} and 3^{rd}, respectively. Black circles represent sample data that has already been collected, and white circles represent sample data that has not yet been collected.

When the time for the control device is 00:00 on the 3^{rd} (time of day T1), A₂, B₂, C₂, D₂ and E₂ are included in the sample data from the past 24 hours. However, when a little time passes and the time of day becomes time of day T2, all that is included in the sample data from the past 24 hours is the data in the shaded triangular region in the figure, that is, only A₃. In such a case, the further a time zone is from GMT + 12 hours, the greater the possibility that the sample data will not be analyzed, meaning that there will be an insufficient number of data sets N and that it will be difficult to always provide reliable analysis results.

FIG. 20(b) is an explanatory diagram showing there being a sufficient number of data sets N when the analysis is based on sample data received in the past 48 hours. When the time for the control devices reaches 00:00 on the 3^{rd} (time of day T1), sample data from analysis devices A through E dated the 1^{st} and 2^{nd} (A₁, B₁, C₁, D₁, E₁; A₂, B₂, C₂, D₂, E₂) are included within the sample data from the past 48 hours. Next, when a little time passes and the time of day becomes time of day T2, the data within the shaded trapezoidal region in the figure (i.e., A₂, B₂, C₂, D₂, and E₂) becomes the population for analysis. In actuality, while the measurement time differs for each analysis device, making the analysis population the sample data of the past 48 hours makes it possible to ensure that there is always a number of sample data sets close to the total number of analysis devices on the system. If there is a plurality of sample data sets from the same analysis device within the population, all such sets'other than the sample data set with the most recent measurement time may be excluded from the analysis.

It should be noted that the reference time for the control device is not limited to GMT + 12 hours. It is also possible to make the period of time subject to analysis longer than 48 hours or shorter than 48 hours; however, 48 hours is expedient in terms of system operations.

### (B-3) Processing Flow

With the exception of the QC processing sub-routine (Step S8 in FIG. 4) performed after receipt of sample data, the processing performed by the control device 1 relating to this embodiment is the same as with the first embodiment. A detailed explanation follows below of the QC processing in this embodiment. The QC processing of this embodiment is divided into (1) today's analysis processing and (2) the previous day's analysis processing.

### (B-3-1) Explanation of Today's Analysis Processing

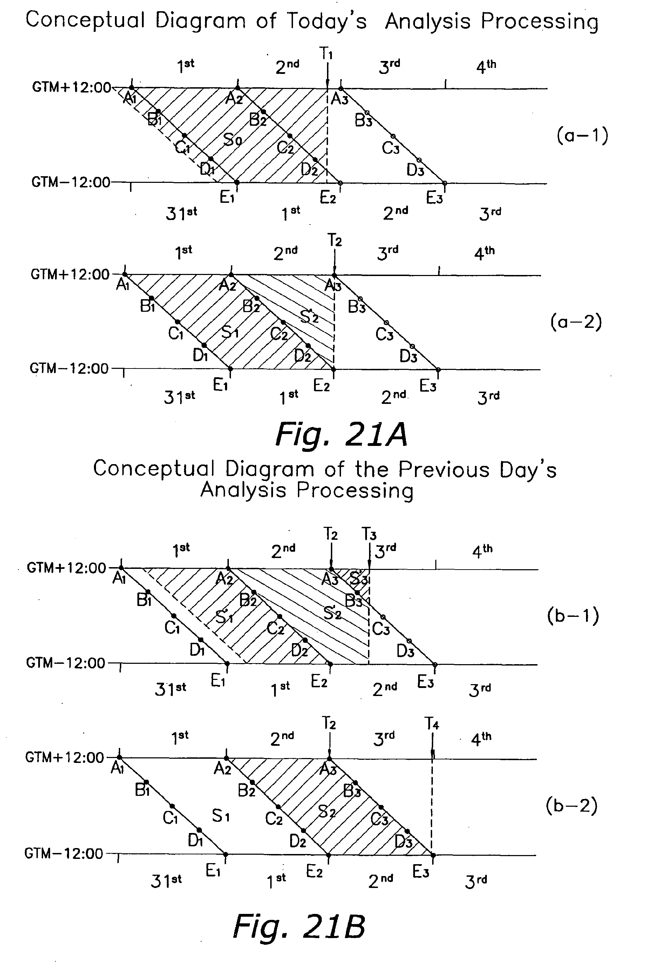

FIG. 21(a) is a drawing explaining the concept of today's analysis processing. In today's analysis processing, first preliminary populations made up sample data dated within the past 48 hours are sequentially created, with the time of the control device 1 as reference. Further more, Sample data analysis is conducted based on the first preliminary population, and today's analysis results are updated. In this embodiment, the update and analysis processing of the first preliminary population is conducted every 10 minutes.

In FIG. 21(a-1), the shaded trapezoidal region S0 shows the first preliminary population at the current time of day T1 (18:00 on the 2^{nd}). With the passage of time the trapezoidal region S0 progresses to the right in the figure. That is, the first preliminary population is updated. As the first preliminary population is updated, the analysis results for today (i.e., the 2^{nd}) are also updated.

At the point of time T2 (00:00 on the 3^{rd}) when the date changes from the 2^{nd} to the 3^{rd}, the previous day's analysis processing for finalizing the analysis results of the second is activated. In FIG. [21](a-2), the shaded trapezoidal region, i.e., the sum of region S1 and S2', represents the first preliminary population at time of day T2. Region S1 represents the group of sample data sets dated the 1^{st} and region S2' represents the group of sample data sets dated the 2^{nd} that were obtained at this point in time.

Even if the previous day's analysis processing has been activated, today's analysis processing continues to be conducted in the same manner as described above. Today's analysis processing, as it continues, updates the analysis results of today (i.e., the 3^{rd}) according to a predetermined timing.

### (B-3-2) Explanation'of the Previous Day's Analysis Processing

FIG. 21(b) explains the previous day's analysis processing. When this processing has been activated at 00:00 on the 3^{rd}, the control device 1 creates a second preliminary population. The control device 1 updates the second preliminary population every 10 minutes, and updates the analysis results for the previous day (i.e., the 2^{nd}) based on the updated second preliminary population.

The creation and update of the second preliminary population is conducted as follows. Every 10 minutes the control device 1 creates a second preliminary population made up of sample data from the past 48 hours. As the time of day progresses from T2 (00:00 on the 3^{rd}), sample data dated today (i.e., the 3^{rd}) that was collected in advanced time zones is deleted from the created second preliminary population.

FIG. 21(b-1) shows a second preliminary population at time of day T3 (10:00 on the 3^{rd}), 10 hours after time of day T2. Region S1' is that group of sample data dated the 1^{st} having an measurement time within 48 hours of T3. Region S2' is a group of sample data with an measurement date of the 2^{nd} that has already been collected. Region S3' is that sample data from the past 48 hours that is dated the 3^{rd} and is to be deleted from the second preliminary population. At time of day T3, the control device computes the analysis results for the previous day (the 2^{nd}) based on the sample data from region S1' and region S2'.

FIG. 21(b-2) is the second preliminary population at the point in time of time of day T4 (00:00 on the 4^{th}), which is 24 hours after time of day T2. The shaded region S2 indicates the second preliminary population at this point in time. The second preliminary population at this point in time comprises the group of sample data sets dated the 2^{nd} from all the analysis devices participating in the remote support system. At this point in time, the control device 1 finalizes the population for the analysis of the day two days prior (the 2^{nd}). The analysis results obtained from this population become the final analysis results for the day two days prior (the 2^{nd}).

### (B-4) Flowchart

In this embodiment, the control device 1 conducts three types of QC processing independently: collection processing, today's analysis processing, and the previous day's analysis processing.

### (B-4-1) Collection Processing

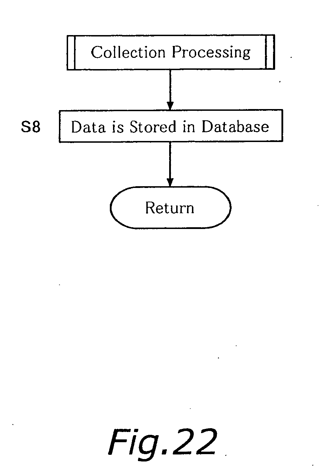

FIG. 22 is the collection processing of sample data that the control device 1 performs. This processing commences when operations proceed to Step S8 (QC processing sub-routine) in the main processing performed by the control device 1 (FIG. 4). In other words, in this embodiment, each time the control device 1 receives sample data, that data is stored in the base database (not shown in figure). The sample data that the control device 1 receives is stored in this base database without any deletions.

### (B-4-2) Today's Analysis Processing

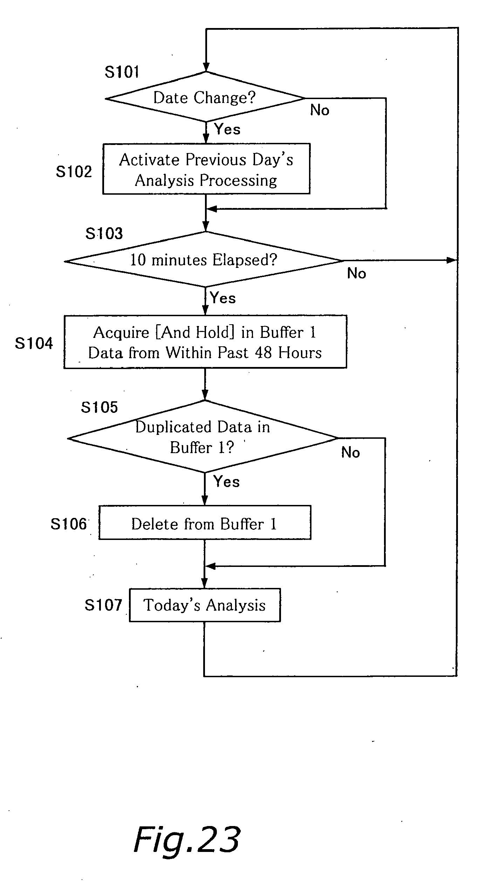

FIG. 23 is a flowchart showing the flow of today's analysis processing performed by the control device 1. In the explanation below, a buffer 1 shall be the work area for forming the first preliminary population that will serve as the basis for today's analysis processing.

Steps S101, S102: The control device 1 determines whether the date has changed (S101). If it has changed, it activates the previous day's processing (S102) (refer to FIG. 21(a-2).

Steps S103, S104, S105, S106: The control device 1 determines whether a predetermined time, i.e., 10 minutes, has elapsed since the previous analysis (S103). If it hasn't elapsed, operations return to Step S101 without analyses being made. If it has elapsed, the first preliminary population is updated and today's analysis is updated.

Specifically, sample data having a time and date within the past 48 hours is first acquired from the base database and is held in the buffer 1 (S104). Next, it is determined whether among the data held in the buffer 1 there is more than one set of data from the same analysis device (S105). If there is, all such data except the most recent is excluded from the buffer 1 (S106) (refer to FIG. 21(a-1).

Step S107: The control device 1 performs analyses based upon the updated first preliminary population. These analysis results will serve as today's analysis results for this point in time.

The control device 1 performs the above processing independently of the sample data collection processing, and updates today's analysis results every 10 minutes, based on sample data from within the past 48 hours.

### (B-4-2) The Previous Day's Analyzing Processing

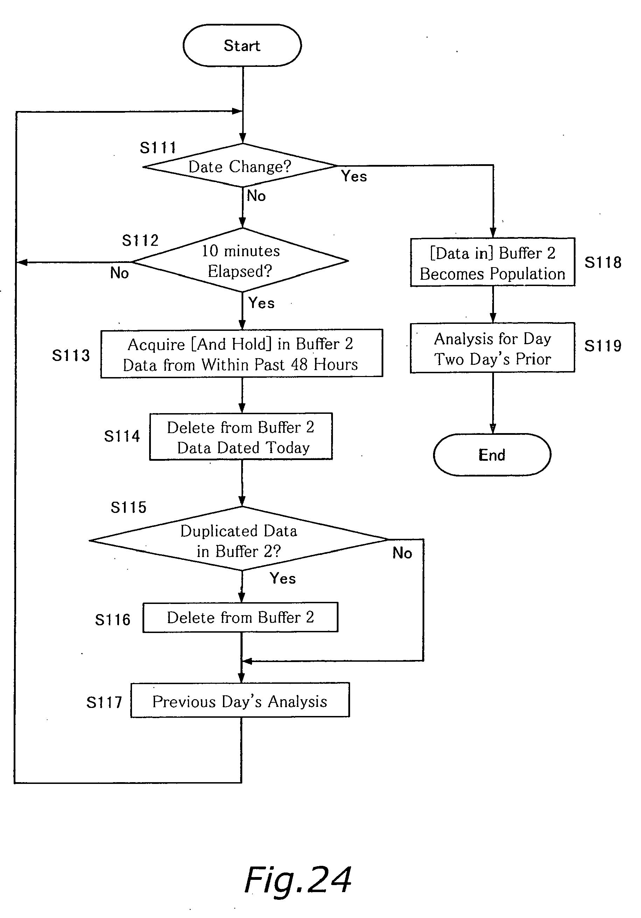

FIG. 24 is a flowchart showing the flow of the previous day's analysis processing that the control device 1 performs. In the explanation below, a buffer 2 shall be the work area for forming the second preliminary population that will serve as the basis for the previous day's analysis processing. When operations in the above-described today's analysis processing proceeds to Step S102, the following processing is activated. As with FIG. 21 above, we will suppose that this processing commenced at time of day T2 (00:00 on the 3^{rd}).

Step S111: The control device 1 again determines whether the date has changed; if it hasn't the processing starting with Step S112 is performed. In other words, until the time of day changes from T3 (00:00 on the 3^{rd}) to T4 (00:00 on the 4^{th}), the update of the second preliminary population and update of the analysis are conducted (S112 to S117, described below). When the time of day reaches 00:00 on the 4^{th}, the analysis results of two days prior, that is, the 2^{nd}, are finalized (Steps 118 through 120 described below).

Step S112: The control device 1 determines whether 10 minutes have elapsed since the previous analysis. If it determines "Yes," then operations proceed to Step S113, and the second preliminary population is updated. If it determines "No," it does not update the preliminary population and operations return to Step S111.

Steps S113, S114, S115, S116: The control device 1 acquires from the base database sample data from within the past 48 hours and holds these in the buffer 2 (S113). Next, the control device 1 deletes data dated today (i.e., the 3^{rd}) from the acquired sample data (S114). Next, it is determined whether among the data held in the buffer 2 there is more than one set of data from the same analysis device (S115). If there is, all such data except the most recent is excluded from the buffer 1 (S116). In this manner, the second preliminary population is updated (refer to FIG. 21(b-1).

Step S117: The control device 1, based on the updated second preliminary population, newly computes analysis results for the previous day, namely, the 2^{nd}. In this manner the analysis results for the 2^{nd} (the previous day) are updated every 10 minutes (S112 to S117).

Step S118: If it is determined at Step S111 that the date has changed, in other words, that the time of day has become 00:00 on the 4^{th}, the control device 1 finalizes the population that will serve as the basis for the analysis results of the 2^{nd}. In other words, the second preliminary population at this point in time becomes the population for the analysis results of two days prior (i.e., the 2^{nd}). Only sample data dated the 2^{nd} is contained in the finalized population (refer to FIG. 21(b-2).

Steps S119: The control device 1 computes the analysis results for two days prior based on the finalized population.

The display of the web page on which the above analysis results are posted is executed based on authentication information input from the analysis device. When a web page is displayed, the control device 1 confirms the time zone of the analysis device. The reason for this is that it is conceivable that the local time in that time zone is a date other the date in the GMT + 12 hours times zone, that is, it is not that date yet. In such cases, the control device 1 does not display today's analysis results for the GMT + 12 hours time zone, but displays only the analysis results of the previous day's analysis processing.

With the above-described processing, based on sample data collected from analysis devices located across the world, today's analysis processing sequentially updates today's analysis results and the previous day's processing updates the previous day's analysis results. In addition, the analysis results for each day are finalized through the previous day's analysis processing. Because the analysis is performed based on there being at least a certain number of sample data sets, the reliability of the analysis results can be improved. Furthermore, because sample data taken from measurements in each time zone is reflected in that day's analysis results, a user can use this system without being aware of any differences in time zones. (C) Storage media on which is recorded the above-described programs of the present invention are included in the present invention. These media can include, among others, computer-readable floppy diskettes, hard disks, semiconductor memory, CD-ROMs, DVDs, and opto-magnetic disks.

(D) Media that transmit the programs of the present invention are also included in the present invention. These transmission media include telecommunication media (optical fibers, wireless networks, et al) in computer network systems (LAN, Internet, wireless communication network) for transporting and supplying program information as carrier.

Through the use of the present invention, the history of an analysis device is stored in a control device, thus making possible rapid response to trouble arising in the analysis device and shortening the down time of the analysis device. Also, the external control of an analysis device can be performed essentially in real time.

While only selected embodiments have been chosen to illustrate the present invention, to those skilled in the art it will be apparent from this disclosure that various changes and modifications can be made herein without departing from the scope of the invention as defined in the appended claims. Furthermore, the foregoing description of the embodiments according to the present invention is provided for illustration only, and not for the purpose of limiting the invention as defined by the appended claims and their equivalents.

## Claims

1. A support method employed in an information terminal connected to analysis devices via a network, the support method comprising:
collecting predetermined historical information showing the operational history of the analysis devices from the analysis devices via the network;
storing collected historical information for each analysis device; and
outputting collected historical information in response to an instruction by the operator of the information terminal.

2. A support method as set forth in claim 1, further comprising:
operating the analysis device from the information terminal via a network.

3. A support method as set forth in claim 1, further comprising:
preparing error determination parameters;
extracting predetermined error information from the historical information;
creating error histories based on the error determination parameters; and
storing correlatively the error histories and the analysis devices.

4. A support method employed in an analysis device connected to a predetermined information terminal via a network, the support method comprising:
transmitting predetermined historical information showing the operational history of the analysis device at a predetermined timing to the information terminal via the network.

5. A support method as set forth in claim 4 further comprising:
accepting operation from a predetermined information terminal via the network.

6. A quality control method employed in an information terminal connected to analysis devices via a network, the quality control method comprising:
receiving sample data that the analysis devices measure predetermined quality control substances via the network;
storing the received sample data;
analyzing the stored sample data for each analysis device and each quality control substance; and
providing the analysis results of the sample data that are received within a predetermined timeframe to the analysis devices.

7. A quality control method employed in analysis devices connected to a predetermined information terminal via a network, the quality control method comprising:
transmitting sample data that predetermined quality control substances are measured to the information terminal via the network;
requesting to the information terminal for analysis results of the sample data;
receiving from the information terminal the analysis results based on the sample data that the information terminal has collected from the analysis devices within a predetermined timeframe; and
outputting the analysis results.

8. A computer-readable storage medium on which is recorded a program for executing the support method employed in an information terminal as set forth in any of claims 1 to 3.

9. A computer-readable storage medium on which is recorded a program for executing the support method employed in an analysis device as set forth in either claim 4 or claim 5.

10. A computer-readable storage medium on which is recorded a program for executing the quality control method employed in an information terminal as set forth in claim 6.

11. A computer-readable storage medium on which is recorded a program for executing the quality control method employed in an analysis device as set forth in claim 7.

12. A control device connected to analysis devices via a network, comprising:
reception means for receiving from the analysis devices predetermined historical information showing the operational history of an analysis device;
storage means for storing historical information for each analysis device; and
output means for outputting historical information in response to an instruction by an operator.

13. An analysis device connected to a predetermined information terminal via a network, comprising transmission means for transmitting predetermined historical information showing operating history to the information terminal via the network according to a predetermined timing.

14. A control device connected to analysis devices via a network, comprising:
reception means for receiving sample data that the analysis devices measure predetermined quality control substances;
storage means for storing received sample data;
analysis means for analyzing the stored sample data for each analysis device and each quality control substance; and
providing means for providing to the analysis device analysis results of sample data received within a predetermined timeframe.

15. An analysis device connected to a predetermined information terminal via a network, comprising:
transmission means for transmitting sample data that predetermined quality control substances are measured to the information terminal via the network;
request means for requesting to the information terminal for analysis results of the sample data;
reception means for receiving from the information terminal the analysis results of the sample data that the information terminal has collected from the analysis devices within a predetermined timeframe; and
output means for outputting received analysis results.
